# EUROPEAN PATENT SPECIFICATION

(11) **EP 4 277 526 B1**
(45) Date of publication and mention of the grant of the patent: **24.07.2024**
(21) Application number: 22700751.5
(22) Date of filing: 11.01.2022
(51) Int. Cl.: A61B 5/145, A61B 5/00

(54) **A SWEAT SENSING APPARATUS**
SCHWEISSERFASSUNGSVORRICHTUNG
APPAREIL DE DÉTECTION DE LA SUEUR

(30) Priority: 13.01.2021 EP 21151253
(43) Date of publication of application: 22.11.2023
(73) Proprietor: Koninklijke Philips N.V., 5656 AG Eindhoven (NL)
(72) Inventor: JOHNSON, Mark Thomas, 5656 AG Eindhoven (NL); VAN GIJSEL, Thomas Johannes, 5656 AG Eindhoven (NL); VAN LIESHOUT, Ron Martinus Laurentius, 5656 AG Eindhoven (NL); GERHARDT, Lutz Christian, 5656 AG Eindhoven (NL)
(74) Representative: Philips Intellectual Property & Standards
(86) International application number: PCT/EP2022/050365
(87) International publication number: WO 2022/152658

(56) References cited:
- EP-A1- 3 649 941
- WO-A1-2017/070640
- US-A1- 2016 287 164
- US-A1- 2018 020 967
- HAIXIA YU ET AL: "Sweat detection theory and fluid driven methods: A review", NANOTECHNOLOGY AND PRECISION ENGINEERING, vol. 3, no. 3, 23 December 2020 (2020-12-23), pages 126 - 140, XP055766759, ISSN: 2589-5540, DOI: 10.1016/j.npe.2020.08.003

## Description

### FIELD OF THE INVENTION

The invention relates to the use of a component to measure a parameter of sweat generated by sweat glands of a subject and, more particularly, to controlling an amount of sweat that can reach the component.

### BACKGROUND OF THE INVENTION

Sweat is a non-obtrusively accessible bio-fluid containing physiologically and metabolically rich information. Sweat contains biomarkers that are indicative of a subject's health and well-being, which can be used for monitoring, amongst other things, dehydration, stress, sleep, children's health and in perioperative monitoring.

The amount of sweat produced by a subject can vary depending on a range of factors, including the health of the subject, whether the subject is exercising, the ambient conditions in which the subject finds themself, or the like. Inaccuracies in measurements of properties of the sweat, such as concentrations of biomarkers contained within the sweat, can occur if too much sweat is produced and is received by a sensor. Furthermore, if too much sweat reaches the sensor, accumulation effects can occur, whereby new sweat is mixed with old sweat. Too little sweat production can lead to, amongst other things, a measurement with a low signal to noise ratio.

Existing techniques are capable of measuring properties of sweat but do not address many of the known issues, such as those discussed above. Existing sweat sensing devices are often used only on people who generate a sufficient amount of sweat (e.g. athletes). However, excessive sweat production can lead to biofouling of sensing components of a sweat sensing device, which can cause damage to the sensing components. This effect is exacerbated when the sensing components are configured to measure relatively lower sweat amounts.

There is therefore a desire for an apparatus that is capable of controlling an amount of sweat, such that it is suitable for use over a range of sweat production rates.

US2018/0020967 discloses sweat sensing devices with excess sweat flow management.

WO2017/070640 discloses devices capable of sample concentration for extended sensing of sweat analytes.

EP3649941 discloses a device, system and method for estimating an analyte concentration in sweat as released by a sweat gland.

US2016/0287164 discloses a portable flow meter for low volume applications.

### SUMMARY OF THE INVENTION

The inventors of the present disclosure have recognised a need for a sweat sensing device that is adapted for measuring a property of sweat (e.g. a concentration of a component of the sweat) both of a person under normal conditions (e.g. a person at rest or carrying on their usual daily activities) and of a person who is producing large amounts of sweat.

According to a first specific aspect, there is provided an apparatus comprising a sweat sensing component for measuring a parameter relating to sweat generated by sweat glands of a subject, a sweat transport channel; a first sweat redirecting component comprising a first responsive material whose form is configured to change in response to a stimulus, so as to redirect sweat and control an amount of sweat able to be transported to the sweat sensing component via the sweat transport channel; and a second sweat redirecting component comprising a second responsive material whose form is configured to change in response to a stimulus, so as to redirect sweat and control an amount of sweat able to be transported to the sweat sensing component via the sweat transport channel, wherein the first sweat redirecting component is positioned at an outlet of the sweat transport channel, such that, in a first configuration, the form of the first responsive material is such that the first sweat redirecting component prevents the passage of sweat through the outlet, and directs the passage of sweat to the sweat sensing component; and, in a second configuration, the form of the first responsive material is such that the first sweat redirecting component permits the passage of sweat through the outlet, and wherein the second sweat redirecting component is positioned at an inlet of the sweat transport channel so as to function as a valve such that, in a first configuration, the form of the second responsive material is such that the second sweat redirecting component prevents the passage of sweat to the sweat sensing component via the sweat transport channel; and, in a second configuration, the form of the second responsive material is such that the second sweat redirecting component permits the passage of sweat to the sweat sensing component via the sweat transport channel.

In some embodiments, the stimulus may comprise an electrical stimulus applied to the first responsive material and/or the second responsive material to bring about a change in the form of the first responsive material and/or the second responsive material, respectively; or a fluidic stimulus to bring about a change in the form of the first responsive material and/or the second responsive material when the fluidic stimulus comes into contact with the first responsive material and/or the second responsive material, respectively. The fluidic stimulus may, for example, comprise a liquid, such as sweat or sweat vapour.

The stimulus may, in some embodiments, comprise an electrical stimulus applied to the first responsive material and/or the second responsive material responsive to at least one of: a parameter value of the sweat measured using the sweat sensing component meeting or exceeding a parameter value threshold; and a sweat rate of sweat from the sweat glands exceeding a sweat rate threshold.

In some embodiments, the first responsive material and/or the second responsive material may comprise at least one material selected from a group comprising: an electroactive polymer; and a piezoelectric material.

The apparatus may, in some embodiments, compromise a sweat rate sensor to measure the sweat rate of sweat from the sweat glands.

In some embodiments, the apparatus may further comprise a processor configured to cause the electrical stimulus to be applied to the first responsive material and/or the second responsive material.

The stimulus may, in some embodiments, comprise a fluidic stimulus to bring about a change in the form of the first responsive material and/or the second responsive material responsive to an amount of sweat contacting the first responsive material and/or the second responsive material exceeding a threshold amount, respectively.

In some embodiments, the first responsive material and/or the second responsive material may comprise a hydrogel.

The parameter relating to sweat may, in some embodiments, comprise a parameter selected from a group comprising: a concentration of a substance in the sweat; and an osmolality of a substance in the sweat.

According to a second specific aspect, there is provided a method of controlling the passage of sweat in an apparatus, the apparatus having a sweat sensing component, a sweat transport channel, a first sweat redirecting component comprising a first responsive material, and a second sweat redirecting component comprising a second responsive material, the method comprising: receiving, at a processor, data indicative of a parameter relating to sweat generated by sweat glands of a subject; and causing an electrical stimulus to be applied to the first responsive material and/or the second responsive material based on the received data; wherein application of the electrical stimulus to the first responsive material causes a form of the first responsive material to change, thereby to redirect sweat and control an amount of sweat able to be transported to the sweat sensing component via the sweat transport channel; wherein application of the electrical stimulus to the second responsive material causes a form of the second responsive material to change, thereby to redirect sweat and control an amount of sweat able to be transported to the sweat sensing component via the sweat transport channel, wherein the first sweat redirecting component is positioned at an outlet of the sweat transport channel, such that, in a first configuration, the form of the first responsive material is such that the first sweat redirecting component prevents the passage of sweat through the outlet, and directs the passage of sweat to the sweat sensing component; and, in a second configuration, the form of the first responsive material is such that the first sweat redirecting component permits the passage of sweat through the outlet, and wherein the second sweat redirecting component is positioned at an inlet of the sweat transport channel so as to function as a valve such that, in a first configuration, the form of the second responsive material is such that the second sweat redirecting component prevents the passage of sweat to the sweat sensing component via the sweat transport channel; and, in a second configuration, the form of the second responsive material is such that the second sweat redirecting component permits the passage of sweat to the sweat sensing component via the sweat transport channel. The method may comprise a computer-implemented method.

In some embodiments, causing an electrical stimulus to be applied to the first responsive material and/or the second responsive material may comprise controlling a power source to supply a current or a voltage to the first responsive material and/or the second responsive material, respectively.

According to a third specific aspect, there is provided a system comprising an apparatus as disclosed herein and a processor configured to cause an electrical stimulus to be applied to the first responsive material and/or the second responsive material.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of an example of a sweat sensing apparatus;
Figs. 2A and 2B are schematic illustrations of a further example of a sweat sensing apparatus;
Figs. 3A and 3B are schematic illustrations of a further example of a sweat sensing apparatus;
Figs. 4A and 4B are schematic illustrations of an example of an electroactive polymer;
Figs. 5A and 5B are schematic illustrations of a further example of an electroactive polymer;
Figs. 6A, 6B, 6C and 6D are schematic illustrations of an example of an electroactive polymer with substrate segments;
Figs. 7A and 7B are schematic illustrations of an example of a sweat redirecting component including a hydrogel;
Fig. 8 is a flowchart of an example of a method of controlling the passage of sweat in an apparatus;
Fig. 9 is a schematic illustration of an example of a processor in communication with a computer-readable medium; and
Fig. 10 is a schematic illustration of an example of a system comprising a sweat sensing apparatus and a processor.

### DETAILED DESCRIPTION OF EMBODIMENTS

Monitoring and analysis of sweat of a subject may provide an insight into the subject's health and well-being. For example, an analysis of biomarkers contained within sweat, which may contain physiologically and metabolically rich information, may reveal information relating to a subject's health status. Biomarkers may include, among others, sodium ions (Na+), chloride ions (Cl+), potassium ions (K+) and the like, which may be used to monitor dehydration; lactate, which may be used as an early warning sign for inflammation and which, in turn, may be used to detect sepsis; glucose, which may be used to detect, among other things, diabetes; and cortisol, which may be used to monitor sleep (e.g. a quality of sleep) and/or stress (e.g. a level of stress) of a subject. As noted above, one known problem with measuring and analysing biomarkers in sweat is that inaccuracies in measurements, or even damage to sensors can result when a sensor is subjected to levels of sweat that are too high. A patch incorporating a sensor may be used to monitor sweat, and the patch may be configured for attachment to a subject. Such patches may be designed for receiving/handling a specific amount of liquid, which can account for both a time element (e.g. how long a device is active or is intended to be used for) and the dynamics (e.g. a sweat flow rate). Designing and optimising patches with respect to microfluidics, waste management, and/or sensing elements to account for the range in sweat amounts can be challenging.

Embodiments of the invention described herein make use of the inventors' recognition that the form or structure (e.g. the shape) of some materials, referred to as responsive materials, can manipulated or modified, either passively or actively, in order to redirect sweat, thereby enabling control of the passage of sweat. Specifically, embodiments relate to an arrangement including a sweat sensing component for measuring a parameter relating to sweat generated by sweat glands of a subject. A sweat redirecting component is formed from, or includes, a material whose form is configured to change in response to a stimulus, so as to redirect sweat and control an amount of sweat able to be transported to the sweat sensing component. In some embodiments, the sweat redirecting component may be referred to as a sweat limiting component. Advantageously, by controlling the amount of sweat able to reach the sweat sensing component, an apparatus or a device incorporating the arrangement can be used with subjects (e.g. people) having a range of sweat production rates, with a reduced chance of the sensitive sensing components being affected by biofouling (e.g. excessive build-up of biofilms that hamper such a device from operating effectively). Embodiments disclosed herein may also help to address the problem associated with an excess of fluid (e.g. sweat) entering a fluid transport system in the device, which could lead to a delayed measurement up-stream (e.g. at the sweat sensing component).

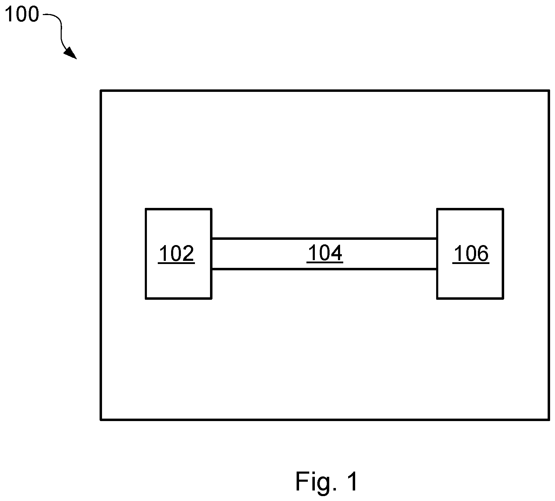

Referring now to the drawings, Fig. 1 is a schematic illustration of an apparatus 100 (e.g. a sweat sensing apparatus). The apparatus comprises a sweat sensing component 102 for measuring a parameter relating to sweat generated by sweat glands of a subject. The sweat sensing component 102 may, for example, comprise a component configured to measure a parameter or characteristic of the sweat itself, such as an amount (e.g. volume) of sweat or a sweat rate (e.g. an amount of sweat per unit time) or a parameter or characteristic of a substance within the sweat, such as a biomarker. For example, the sweat sensing component 102 may be configured to measure a concentration of a particular substance in the sweat.

The apparatus 100 also comprises a sweat transport channel 104. The sweat transport channel 104 is configured to transport sweat to the sweat sensing component 102, for example from an inlet or entrance of the apparatus 100. In some embodiments, for example, the sweat transport channel 104 may be configured to transport sweat from a surface of the subject (e.g. the subject's skin) through the apparatus 100 to the sweat sensing component 102. The sweat transport channel 104 may comprise one or more channels, pipes, tubes or conduits suitable for the passage of fluids, such as a sweat, and may have any cross-sectional shape (e.g. circular, rectangular, or the like). One or more other components (e.g. electrowetting components) may be provided to assist with transporting sweat through the sweat transport channel 104, but are not discussed further in this disclosure.

The apparatus 100 also comprises a sweat redirecting component 106 (which may be referred to as a first sweat redirecting component). The sweat redirecting component 106 comprises (e.g. is formed of, or includes) a responsive material whose form is configured to change in response to a stimulus, so as to redirect sweat and control an amount of sweat able to be transported to the sweat sensing component via the sweat transport channel. As used herein, the term "responsive material" is intended to refer to a material capable of responding to an input or stimulus. The response of the material may comprise a change in one or more of its structure, form, shape, size, or the like. The first sweat redirecting component 106 is positioned at an outlet of the sweat transport channel 104 such that, in a first form or configuration, redirect the sweat so as to restrict the passage of sweat via the sweat transport channel 104 to the sweat sensing component 102 and, in a second form or configuration, allow the passage of sweat via the sweat transport channel to the sweat sensing component. By redirecting the sweat before it enters the apparatus 100, or within the apparatus, the amount of sweat able to reach certain regions of the apparatus (e.g. the sweat sensing component 102) can be controlled. For example, the amount of sweat able to be transported to the sweat sensing component 102 may be restricted or limited (e.g. by directing sweat away from the sweat sensing component) under certain circumstances, such as the amount of sweat exceeding a threshold amount, or parameter or characteristic of the sweat (e.g. pH level indicating how acidic or basic the sweat it, or a concentration of a component of the sweat) meeting a particular criterion. The stimulus used to bring about the change in the form of the responsive material may comprise an electrical stimulus or a fluidic stimulus, and these stimuli are discussed in greater detail below.

Depending on the position of the sweat redirecting component 106 relative to the apparatus 100, the passage of sweat may be redirected at different points in the apparatus or in the sweat transport channel 104. The sweat redirecting component 106 (which may be referred to as a first sweat redirecting component) is configured or positioned at an outlet of the sweat transport channel 104 such that sweat is able to enter the apparatus 100 (e.g. within the sweat transport channel 104), but an amount of sweat able to reach the sweat sensing component 102 may be limited. In such embodiments, the sweat redirecting component 106 may be configured to redirect sweat within the apparatus 100 or direct the passage of sweat away from the sweat sensing component or out of the apparatus.

The apparatus 100 also comprises a second sweat redirecting component 107 comprising a responsive material (e.g. a second responsive material). The second sweat redirecting component 107 is configured or positioned to control (e.g. limit or prevent) an amount of sweat from entering apparatus 100, and thus control an amount of sweat reaching the sweat sensing component. For example, the second sweat redirecting component 107 may act as a gate or valve preventing sweat from entering or restricting the amount of sweat entering the apparatus 100 by redirecting the incoming sweat..

Advantageously, the sweat redirecting component 106 and/or the second sweat redirecting component 107 may control an amount of sweat encountering the sweat sensing component such that the sweat sensing component is able to operate effectively (e.g. to allow optimisation of the sweat sensing component by helping to ensure that an optimum amount of sweat is able to reach the sweat sensing component). Various embodiments are now described in greater detail.

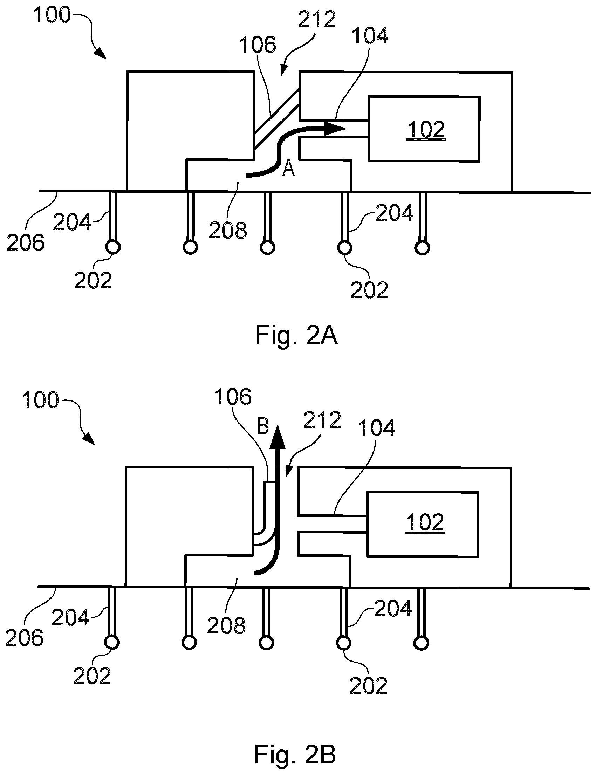

Figs. 2A and 2B are schematic illustrations of an example of the apparatus 100 according to a specific embodiment. The apparatus 100 includes a sweat sensing component 102 and a sweat transport channel 104 in fluidic communication with the sweat sensing component. In this example, the sweat transport channel 104 comprises a single, straight channel. However, it will be appreciated that, in other examples, multiple channels may be provided through which sweat may be transported to reach the sweat sensing component 102. The apparatus 100 also includes a sweat redirecting component 106 which, in this embodiment, is positioned within the apparatus 100. Sweat generated by a sweat gland 202 of a subject travels up a sweat duct 204 to a surface of the skin 206 of the subject. Sweat may be received in a sweat receiving area 208 of the apparatus 100, and may be transported along the sweat transport channel 104 to the sweat sensing component 102. A fluid transport mechanism (not shown) may be provided to aid the movement of sweat from the surface of the skin 206, through the sweat receiving area 208 and/or along the sweat transport channel 104.

In this example, the sweat redirecting component 106 comprises a closure movable between a first configuration (e.g. a closed position) and a second configuration (e.g. an open position). In Fig. 2A, the sweat redirecting component 106 is in the first configuration (e.g. the closed position) such that sweat is directed towards the sweat sensing component 102 via the sweat transport channel 104, as indicated by arrow A. When the sweat redirecting component 106 is in the closed position, sweat may be prevented from exiting the apparatus 100 via an exhaust or vent 212 by redirecting the sweat away from the exhaust or vent.

In Fig. 2B, the sweat redirecting component 106 is in the second configuration (e.g. the open position). In this configuration, an amount of sweat may travel from the surface of the skin 206, through the sweat receiving area 208, and through the exhaust or vent 212, as indicated by arrow B. In some embodiments, a proportion of the sweat entering the apparatus 100 may travel through the exhaust 212 while a proportion of the sweat travels along the sweat transport channel 104 to the sweat sensing component 102. The passage leading from the sweat receiving area 208 to the exhaust or vent 212 may be considered part of the sweat transport channel 104, such that the exhaust or vent may comprise an outlet of the sweat transport channel. The structure and/or arrangement of components in the apparatus 100 may determine the proportion of sweat that is able to travel along the sweat transport channel 104 to the sweat sensing component 102 and the proportion of sweat that exits the apparatus via the exhaust 212. For example, the sweat transport channel 104 may have a relatively smaller cross-sectional area than the cross-sectional area associated with the exhaust 212, which may lead, in some examples, to a relatively larger amount of sweat flowing through the exhaust than through the sweat transport channel. In any case, however, when the sweat redirecting component 106 is in the open position, the amount of sweat reaching the sweat sensing component 102 is less than when the sweat redirecting component is in the closed position (as in Fig. 2A). In the arrangement shown in Fig. 2B, therefore, the form of the responsive material of the sweat redirecting component 106 may be such that the sweat redirecting component permits the passage of sweat through an outlet (e.g. the exhaust 212). An effect of the sweat redirecting component 106 being in an open position is that sweat, sweat vapour or other fluid may be exhausted or vented from the apparatus 100 rather than overwhelming the sweat sensing component 102 (e.g. avoiding biofouling of the sweat sensing component). Thus, according to some embodiments, such as those shown in Figs. 2A and 2B, the sweat redirecting component 106 may be positioned in the sweat transport channel 104 (e.g. at or near to an outlet of the sweat transport channel 104), such that, in a first configuration (e.g. a closed position as shown in Fig. 2A), the form of the responsive material is such that the sweat redirecting component prevents the passage of sweat through the outlet and through the exhaust 212, and directs the passage of sweat to the sweat sensing component 102; and, in a second configuration (e.g. an open position as shown in Fig. 2B), the form of the responsive material is such that the sweat redirecting component permits the passage of sweat through the outlet and through the exhaust 212.

The sweat redirecting component 106 is positioned at an outlet of the sweat transport channel 104. This position (i.e. the position of the sweat redirecting component 106) includes that the sweat redirecting component may be located somewhere between the outlet (e.g. the exhaust 212) and the sweat sensing component 102. An advantage of the sweat redirecting component being located between the outlet and the sweat sensing component is that the sweat redirecting component may be configured (e.g. see the configuration in Fig. 2B) to allow an amount of sweat (e.g. a defined amount of sweat) to pass beyond (e.g. continue through the sweat transport channel towards the exhaust) the sweat sensing component and escape (e.g. be vented from) the sweat sensing device 100. In this configuration (e.g. the configuration shown in Fig. 2B), the sweat redirecting component may be configured to not stop sweat reaching the sweat sensing component (e.g. allow passage of a defined amount of sweat to the sweat sensing component 102), while venting any excess sweat from the sweat sensing device 100. In this way, excess sweat may be vented from the sweat sensing device thereby avoiding biofouling of the sweat sensing component 102, while a defined amount of sweat may still be allowed to travel to the sweat sensing component for measuring a property of the sweat. A further advantage of the sweat redirecting component 106 being located between the outlet and the sweat sensing component 102 is that the amount of sweat allowed to travel to any other components of the sweat sensing device 100 downstream of the sweat redirecting component may be controlled.

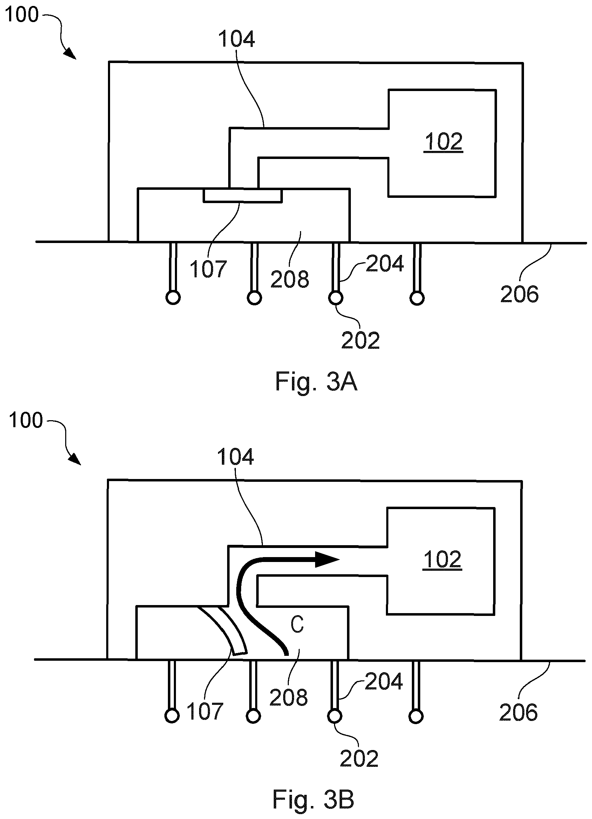

An alternative embodiment is shown in Figs. 3A and 3B, in which an apparatus 100 comprises both a sweat redirecting component 106 (not shown) and a second sweat redirecting component 107. The second sweat redirecting component 107 functions as a closure or a valve to prevent or allow sweat to move into the apparatus 100 and/or the sweat transport channel 104, rather than functioning as a closure of a vent or exhaust, as per the sweat redirecting component 106 (e.g. the first sweat redirecting component) shown in Figs. 2A and 2B.

Fig. 3A is a schematic illustration of an example of the apparatus 100 including the sweat sensing component 102, the sweat transport channel 104, and the second sweat redirecting component 107. The second sweat redirecting component 107 again comprises (e.g. is formed of or includes) a responsive material (e.g. a second responsive material) whose form is configured to change in response to a stimulus, so as to redirect sweat and control an amount of sweat able to be transported to the sweat sensing component via the sweat transport channel. In this example, the second sweat redirecting component 107 is positioned either at inlet of the sweat transport channel 104 or at an inlet or opening of the apparatus 100, through which sweat may pass to reach the sweat transport channel and/or the sweat sensing component 102. In the arrangement shown in Fig. 3A, the second sweat redirecting component 107 is in a first configuration (e.g. a closed position) blocking the inlet, and thereby preventing the movement of sweat into the sweat transport channel 104.

In Fig. 3B, the second sweat redirecting component 107 is in a second configuration (e.g. an open position). In this configuration, the form of the second responsive material (e.g. the responsive material associated with the second sweat redirecting component) has changed such that the second sweat redirecting component 107 permits the passage of sweat to the sweat sensing component 102 via the sweat transport channel 104 or to the sweat transport channel itself, in the direction of the arrow C.

Thus, as those shown in Figs. 3A and 3B, the second sweat redirecting component 107 is positioned at an inlet of the sweat transport channel 104 so as to function as a valve such that, in a first configuration (e.g. a closed position as shown in Fig. 3A), the form of the second responsive material is such that the second sweat redirecting component prevents the passage of sweat to the sweat sensing component 102 via the sweat transport channel; and, in a second configuration (e.g. an open position as shown in Fig. 3B), the form of the second responsive material is such that the second sweat redirecting component permits the passage of sweat to the sweat sensing component via the sweat transport channel.

Figs. 2 and 3 show just two examples of the many possible arrangements of the sweat redirecting component 106 and/or the second sweat redirecting component 107 that could be implemented to permit and restrict the passage of sweat to the sweat sensing component 102 of the apparatus 100 by redirecting the sweat appropriately. As noted above, the sweat redirecting component 106 comprises a responsive material whose form can change in response to a stimulus. Similarly, the second sweat redirecting component 107 comprises a responsive material (e.g. a second responsive material) whose form can change in response to a stimulus. The stimulus that causes the form of the responsive material and/or second response material to change may, in some embodiments, comprise an electrical stimulus or a fluidic stimulus. In some embodiments, the stimulus may comprise an electrical stimulus applied to the responsive material to bring about a change in the form of the responsive material. This may be referred to as an active stimulus, as the stimulus is applied to the responsive material actively by a controller. In other embodiments, the stimulus may comprise a fluidic stimulus to bring about a change in the form of the responsive material when the fluidic stimulus comes into contact with the responsive material. This may be referred to as a passive stimulus, as the stimulus causes the response of the responsive material automatically when contacting the responsive material. Embodiments discussed herein (such as those discussed above with reference to Figs. 2 and 3) may incorporate one or more of the electric stimulus and the fluidic stimulus.

A responsive material that is responsive to an electrical stimulus may have a form that changes automatically when a voltage or current is applied to it or across it. Several materials are known to change form when a voltage or current is applied to them. According to some examples, the responsive material may comprise at least one material selected from a group comprising: an electroactive polymer; and a piezoelectric material.

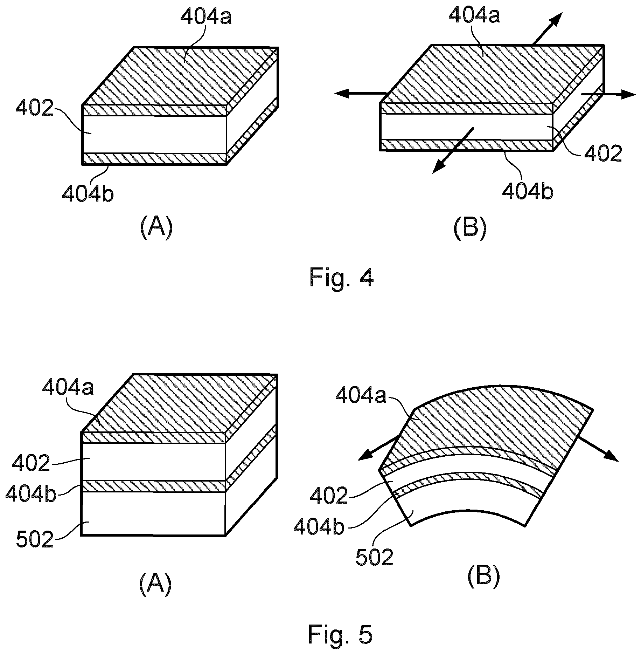

An electroactive polymer (EAP) is an example of a type of responsive material that is responsive to an electrical stimulus. Figs. 4 and 5 are schematic illustrations of examples of part of a sweat redirecting component 106 (or, equivalently, a second sweat redirecting component 107) that includes an electroactive polymer. In the example shown in Fig. 4A, an electroactive polymer 402 is sandwiched between a pair of flexible electrodes 404a, 404b. In this example, when a voltage is applied between the electrodes 404a, 404b, the electroactive polymer 402 is caused to expand in the directions indicated by the arrows in Fig. 4B. Thus, if such a sweat redirecting component 106, or a second sweat redirecting component 107, were positioned across a channel (e.g. sweat transport channel 104) or across an inlet or outlet of the channel or the apparatus 100, then access past the sweat redirecting component and/or the second sweat redirecting component can be controlled by applying or removing a voltage across the electrodes 404a, 404b. For example, when a voltage is applied across the electrodes 404a, 404b, the electroactive polymer 402 would be caused to expand, thereby covering or blocking the channel, inlet or outlet.

In the example shown in Fig. 5, a structure is formed by the electroactive polymer 402 and the electrodes 404a, 404b being mounted on a carrier layer 502. When a voltage is applied across the electrodes 404a, 404b, the carrier layer 502 is caused to bend or flex, along with the electroactive polymer 402 and the electrodes, in the direction indicated by the arrows in Fig. 5B. In this way, the application of a voltage across the electrodes 404a, 404b would cause the structure to bend or flex to block the channel, inlet or outlet, or to bend or flex in order to create an opening through the channel, inlet or outlet. Electroactive polymers are associated with a number of advantages, including that they can operate using a relatively low power consumption, they can have a small form factor, they may be flexible, they may be noiseless or generate relatively low levels of noise, and they may have a relatively fast response time (e.g. they change form quickly following application of an electrical stimulus).

As noted above, the electrical stimulus may be provided to the responsive material in the form of a current and/or a voltage being supplied to the sweat redirecting component 106 (e.g. to electrodes associated with the responsive material) and/or to the second sweat redirecting component 107 (e.g. to electrodes associated with the second responsive material). The current and/or voltage may be supplied by a power source (e.g. a battery or a mains power supply), which may form a part of the apparatus 100 or may be external to the apparatus. The application of the current or voltage may be controlled by a processor which may form part of the apparatus 100 or may be in communication with one or more components associated with the apparatus, including the power supply. Electroactive polymers may comprise field driven materials (e.g. electrostrictive polymers including dielectric elastomers, PVDF based relaxor polymers and liquid crystal elastomers (LCE)) or ionic driven materials (e.g. conjugated polymers, carbon nanotube (CNT) - polymer composites and Ionic Polymer Metal Composites (IPMC)). Field driven electroactive polymers may be actuated by an electric field through direct electromechanical coupling while the actuation mechanism for ionic electroactive polymers may involve diffusion of ions and/or electrochemical oxidation and reduction. An electric field in diffusion driven ionic polymer actuators may be confined to an electric double layer and thus may be independent of the thickness of the actuator itself. Such ionic polymer actuators may show little hysteresis and low sustain current (e.g. much less than an actuation current). In other embodiments, the responsive material may comprise a piezoelectric material, which may function in a similar way; a voltage or current applied to the piezoelectric material may cause the structure or form of the material and any carrier layer or material attached to it to change (e.g. expand or bend) to block access to or permit access to a channel, an inlet or an outlet.

The application of a current or voltage to the responsive material (and equivalently, the second responsive material) may be based on a measurement made by a sensor and, in some embodiments, such a sensor may form part of the apparatus 100. In some embodiments, an electrical stimulus may be applied to the responsive material in response to a parameter value of the sweat measured using a sweat sensing component 102 (e.g. a biosensor) meeting or exceeding a parameter value threshold. In other words, a feedback loop may be employed, such that an electrical stimulus is applied to a responsive material based on a measurement of the sweat sensing component. For example, the sweat sensing component might be configured to measure a parameter such as a concentration or osmolality of a substance in the sweat (e.g. sodium ions, potassium ions, lactate, or the like) and, if the concentration or osmolality of the substance meets or exceeds a defined threshold, then a current or voltage may be applied to the responsive material to bring about a change in its form. Thus, the parameter relating to sweat (i.e. the parameter measured by the sweat sensing component 102) may comprise a parameter selected from a group comprising: a concentration of a substance in the sweat; and an osmolality of a substance in the sweat. In this way, if it is determined that the sweat sensing component 102 is receiving sweat containing too much of a particular substance (e.g. a substance that could damage the sweat sensing component), then the current or voltage may be applied to the responsive material in order to cause the sweat redirecting component 106 to expand or bend (or otherwise change its form) to at least partially block a channel or inlet, to redirect sweat and control the amount of sweat able to reach the sweat sensing component, or to at least partially unblock an outlet, to enable sweat to be vented from the apparatus 100 without reaching the sweat sensing component.

In some embodiments, an electrical stimulus may be applied to the responsive material and/or the second responsive material in response to a parameter value of the sweat measured using a sweat sensing component 102 (e.g. a biosensor) meeting or exceeding a parameter value threshold. In this way, if it is determined that the sweat sensing component 102 is receiving sweat containing too much of a particular substance (e.g. a substance that could damage the sweat sensing component), then the current or voltage may be applied to the responsive material and/or the second responsive material in order to cause the sweat redirecting component 106 and/or the second sweat redirecting component, respectively, to expand or bend (or otherwise change its form) to at least partially block a channel or inlet, to redirect sweat and control the amount of sweat able to reach the sweat sensing component, or to at least partially unblock an outlet, to enable sweat to be vented from the apparatus 100 without reaching the sweat sensing component. Advantageously, by causing both the sweat redirecting component 106 and the second sweat redirecting component to redirect sweat, the amount of sweat reaching the sweat sensing component may be controlled more precisely. As a result, the likelihood of biofouling of the sweat sensing component is further reduced.

In other embodiments, an electrical stimulus may be applied to the responsive material and/or the second responsive material in response to a sweat rate of sweat from the sweat glands exceeding a sweat rate threshold. In some embodiments, the electrical stimulus may be applied in response to a combination of a concentration or osmolality of a substance in the sweat and a sweat rate exceeding a combined threshold value. The sweat rate may be measured using the sweat sensing component 102 itself or using a separate component capable of measuring sweat rate (e.g. a sweat rate sensor or a flow rate sensor). Such a sweat rate sensor or a flow rate sensor may form part of the apparatus 100 or may comprise a separate component in communication with the processor controlling the application of the current or voltage to the responsive material and/or the second responsive material. Thus, the apparatus 100 may further comprise a sweat rate sensor to measure the sweat rate of sweat from the sweat glands. In some examples, a galvanic skin response sensor may be used to determine the sweat rate. In some embodiments, the apparatus 100 may further comprise a processor configured to cause the electrical stimulus to be applied to the responsive material and/or the second responsive material. The processor may also be configured to receive data indicative of the measured sweat rate from the sweat rate sensor. As noted above, the processor and/or the sweat rate sensor may alternatively comprise separate components.

As discussed in greater detail below, the responsive material and/or the second responsive material may, in some embodiments, respond automatically to the presence of a liquid, such as sweat, or to the presence of a liquid having a particular parameter or characteristic, such as a liquid containing a minimum concentration or osmolality of a particular component (e.g. ions), or a liquid having a particular pH level. In this way, the sweat intake can be linked to the sweat physiology. Thus, the sweat redirecting component 106 and/or the second swear redirecting component 107 enables dynamic and adaptive redirection of sweat from the subject in response to properties of the sweat, which may change overtime.

A responsive material that is responsive to a fluidic stimulus may have a form that changes automatically when it comes into contact with particular fluids, such as a liquid (e.g. sweat). In some embodiments, the stimulus may comprise a fluidic stimulus to bring about a change in the form of the responsive material and/or the second responsive material responsive, for example, to an amount of sweat contacting the responsive material and/or the second responsive material exceeding a threshold amount. The responsive material and/or the second responsive material may, in some embodiments, comprise a hydrogel. A hydrogel is an example of a responsive material, or reactive material, whose physical properties (e.g. volume) may change as a result of changes in the chemistry of the environment in which the hydrogel is located (e.g. changes in pH, temperature, humidity, ionic strength, concentration of specific drugs or chemicals, or the like). A hydrogel may change its physical properties (e.g. swell, shrink or bend) dependent on the balance between the polymer-water Gibbs energy, which may be associated with an elastic nature of a hydrogel polymer network. Another approach to induce a specific volume response is known as molecular imprinting. A hydrogel may, for example, be used in embodiments in which a relatively small amount of sweat is produced (e.g. a low sweat rate).

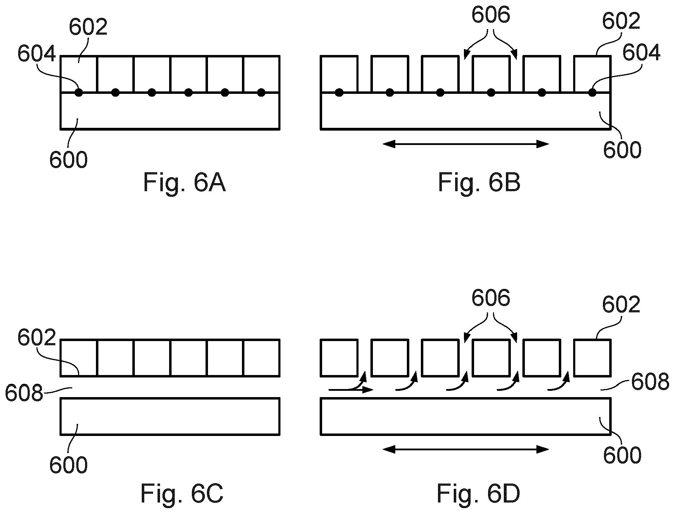

Figs. 6A to 6D are schematic illustrations showing a further example of a sweat redirecting component 106 and/or a second sweat redirecting component 107. In this example, sweat redirecting component 106 and/or second sweat redirecting component 107comprises an electroactive polymer 600 attached or coupled to a plurality of substrate segments 602 at coupling or attachment positions 604. Figs. 6A and 6C show the electroactive polymer 600 in an unactuated state, where no stimulus (e.g. an electrical stimulus) has been applied. Figs. 6B and 6D show the electroactive polymer 600 in an actuated state, where a stimulus (e.g. an electrical stimulus) has been applied. When a stimulus is applied to the electroactive polymer 600, the electroactive polymer changes form (e.g. stretches, changes curvature, or the like). As the electroactive polymer 600 changes form in response to the stimulus, the substrate segments 602 are also caused to move. In this example, as the electroactive polymer 600 expands in the directions by the arrows in Figs. 6A and 6D, the substrate segments 602 are caused to separate from one another, such that gaps or openings 606 are formed between adjacent substrate segments, through which fluid, such as sweat, may flow. Figs. 6C and 6D show a channel 608 between the electroactive polymer 600 and the layer of substrate segments 602. Thus, when the electroactive polymer 600 is in an unactuated state (e.g. as in Fig. 6C), then sweat may flow through the channel 608, towards the sweat transport channel 104 and/or the sweat sensing component 102. However, when the electroactive polymer 600 is in an actuated state (e.g. as in Fig. 6D), then sweat may exit the channel 608 via the openings 606, and the amount of sweat able to reach the sweat sensing component 102 may be reduced.

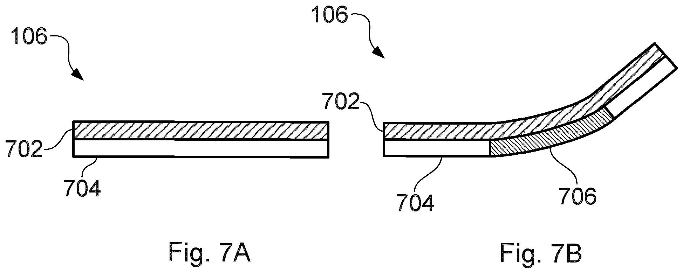

Fig. 7 is a schematic illustration of an example of a sweat redirecting component 106 and/or a second sweat redirecting component 107 comprising a hydrogel which may change form (e.g. shape) in response to encountering a fluid (e.g. sweat, water, or the like). The sweat redirecting component 106 and/or the second sweat redirecting component 107 in this example includes a non-absorbent layer (e.g. a polymer layer) 702 coupled or attached to a layer of hydrogel material 704. In Fig. 7A, the hydrogel material 704 is dry, or is not in contact with sufficient amounts of a liquid needed to bring about a change of form of the hydrogel material, and both the hydrogel material and the non-absorbent layer 702 are in a relatively straight or flat (e.g. not bent) form. In Fig. 7B, a portion 706 of the hydrogel material 704 has come into contact with a liquid (e.g. sweat), thereby causing this portion of the hydrogel material to change form by contracting or bending upwards. Thus, both the hydrogel material 704 and the non-absorbent layer 702 are caused to bend. In use, such a sweat redirecting component 106 is positioned at an outlet of the sweat transport channel 104. When the hydrogel material 704 is relatively dry, or has not encountered or absorbed sufficient amounts of sweat, then the sweat redirecting component 106 may block the outlet, thereby preventing sweat from being vented from the apparatus 100, and directing it towards the sweat sensing component 102, as in the example shown in Fig. 2A. If the amount of sweat encountering the hydrogel material 704 increases to a sufficient level, then the hydrogel material and, therefore, the sweat redirecting component 106, may be caused to bend, thereby opening the vent, and allowing sweat to exit the apparatus 100 via the outlet, as in the example shown in Fig. 2B. Advantageously, excess sweat produced by the subject, which can lead to biofouling of sensitive sensing components, can be exhausted from the apparatus 100 before it reaches the sweat sensing component(s) 102. Alternatively, if the amount of sweat being produced by the subject is relatively low, then a higher proportion, if not all, of the generated sweat may be directed to the sweat sensing component(s) 102 of the apparatus 100.

In another example, a sweat redirecting component 107 comprising a hydrogel material 704 is positioned at an inlet of the apparatus 100 or the sweat transport channel 104, similar to the arrangement shown in Figs. 3A and 3B. The sweat redirecting component 107 may be positioned such that, when mounted on or attached to the subject, the hydrogel material 704 of the sweat redirecting component 107 is in contact with the skin of the subject. In such an example, if low amounts of sweat are produced by the subject, then the hydrogel material 704 may remain flat (e.g. in the configuration shown in Fig. 7A) while, if large amounts of sweat are produced, then the hydrogel material may absorb enough sweat to cause the sweat redirecting component 107 to bend into a position that at least partially blocks the inlet, thereby reducing the amount of sweat able to enter the apparatus 100 and reach the sweat sensing component 102. When the amount of sweat generated by the subject reduces to an acceptable level, the hydrogel material 704 may return to its flat configuration (Fig. 7A), thereby allowing more sweat to enter the apparatus 100 and reach the sweat sensing component 102.

A further advantage of using a responsive material in the sweat redirecting component 106 and/or the second sweat redirecting component 107 is that the sweat redirecting component 106 and/or the second sweat redirecting component 107 can be much more compact than a traditional mechanical valve. Thus, the apparatus 100 may have a relatively small form factor, which may be more comfortable and convenient for a subject to wear on their skin than an apparatus that uses mechanical valves to stop sweat from entering the apparatus.

Since sweat is redirected away from the sweat sensing component 102 when needed, most components of the apparatus 100 need not absorb excess sweat and, therefore, the apparatus of the present disclosure does not become saturated by sweat. Thus, the apparatus 100 will not stop working or work less effectively once a threshold amount of sweat has been received. Even in embodiments in which a hydrogel is used (e.g. Fig. 7), only a portion of the sweat redirecting component 106 and/or the second sweat redirecting component 107 absorbs sweat (i.e. the hydrogel material 704). The non-absorbent layer 702 does not absorb sweat, and still acts to redirect sweat.

Embodiments in which the responsive material responds to a fluidic stimulus have a further advantage in that an electrical input is not required in order for the sweat redirecting component 106 and/or the second sweat redirecting component 107 to operate. Electrical connections are therefore not needed for the sweat redirecting component 106 and/or the second sweat redirecting component 107, meaning the apparatus 100 is more energy efficient, and the construction of the apparatus is less complex than apparatuses using electrical connections.

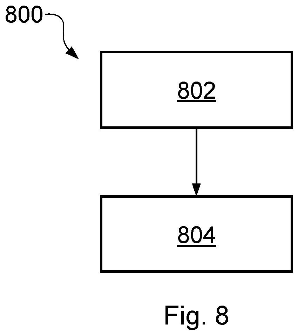

According to a second aspect of the disclosure, a method is provided. Fig. 8 is a flowchart of an example of a method 800. The method 800, which may comprise computer-implemented method, may comprise a method of controlling the passage of sweat in an apparatus (e.g. the apparatus 100). The apparatus has a sweat sensing component 102, a sweat transport channel 104 and a sweat redirecting component 106 comprising a responsive material. The method 800 comprises, at step 802, receiving, at a processor, data indicative of a parameter relating to sweat generated by sweat glands of a subject. At step 804, the method 800 comprises causing an electrical stimulus to be applied to the responsive material based on the received data. In some embodiments, the application of the electrical stimulus to the responsive material causes a form of the responsive material to change, thereby to redirect sweat and control an amount of sweat able to be transported to the sweat sensing component 102 via the sweat transport channel 104. In other embodiments, the application of the electrical stimulus to the responsive material may cause a form of the responsive material to change, thereby to allow an amount of sweat able to be transported to the sweat sensing component 102 via the sweat transport channel 104. The sweat redirecting component 106 is positioned at an outlet of the sweat transport channel, such that, in a first configuration, the form of the responsive material is such that the sweat redirecting component prevents the passage of sweat through the outlet, and directs the passage of sweat to the sweat sensing component; and, in a second configuration, the form of the responsive material is such that the sweat redirecting component permits the passage of sweat through the outlet. The data indicative of a parameter relating to sweat may, for example, comprise a sweat rate, a concentration or osmolality of a substance in the sweat, a volume of sweat, or the like. The data may be received from a sensor, such as the sweat sensing component 102 discussed above. In some embodiments, causing an electrical stimulus to be applied to the responsive material may comprise controlling a power source to supply a current or a voltage to the responsive material. For example, if it is determined that the sweat rate exceeds a defined threshold, then the processor may control the power source to supply a current or voltage to the responsive material, in order to cause the sweat redirecting component to move into a position where the movement of sweat towards the sweat sensing component 102 is restricted.

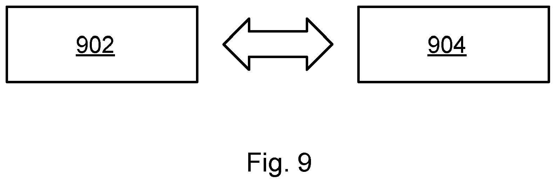

Fig. 9 is a schematic illustration of an example of a processor 902 in communication with a computer-readable medium 904. A computer program product comprises a non-transitory computer-readable medium 904, the computer-readable medium having computer-readable code embodied therein, the computer-readable code being configured such that, on execution by a suitable computer or processor 902, the computer or processor is caused to perform steps of the method 800 discussed herein.

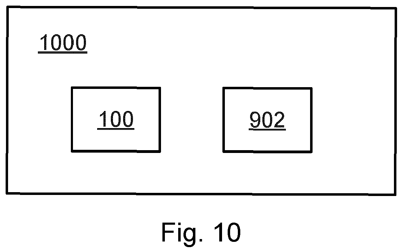

According to a third aspect of the disclosure, a system is provided. While, in some embodiments, the apparatus 100 may comprise a processor configured to perform steps of the method 800, the processor may, in other embodiments, be a separate component. Fig. 10 is a schematic illustration of an example of a system 1000. The system 1000 comprises the apparatus 100 and a processor (e.g. the processor 902) configured to cause an electrical stimulus to be applied to the responsive material. The processor 902 may communicate with components of the apparatus 100 (e.g. the sweat sensing component 102) via a wired or wireless communication protocol. In some embodiments, the apparatus 100 may itself comprise a processor configured to communicate with the processor 902.

The embodiments described above provide a mechanism by which the amount of sweat able to reach a sweat sensing component of an apparatus can be controlled, so that apparatuses can be used for a range of people who generate different amounts of sweat. The apparatuses disclosed herein can therefore be considered to be more versatile than known apparatuses.

The processor 902 can comprise one or more processors, processing units, multi-core processors or modules that are configured or programmed to control the apparatus 100 in the manner described herein. In particular implementations, the processor 902 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An example relating to a computer program product comprises computer-executable instructions corresponding to each processing stage of at least one of the methods set forth herein. These instructions may be sub-divided into sub-routines and/or stored in one or more files that may be linked statically or dynamically. Another example relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (100) comprising:
a sweat sensing component (102) for measuring a parameter relating to sweat generated by sweat glands of a subject;
a sweat transport channel (104); and
a first sweat redirecting component (106) comprising a first responsive material whose form is configured to change in response to a stimulus, so as to redirect sweat and control an amount of sweat able to be transported to the sweat sensing component (102) via the sweat transport channel (104);
wherein the first sweat redirecting component (106) is positioned at an outlet of the sweat transport channel (104), such that, in a first configuration, the form of the first responsive material is such that the first sweat redirecting component (106) prevents the passage of sweat through the outlet, and directs the passage of sweat to the sweat sensing component (102); and, in a second configuration, the form of the first responsive material is such that the first sweat redirecting component (106) permits the passage of sweat through the outlet, **characterized in that**:
the apparatus comprises a second sweat redirecting component (107) comprising a second responsive material whose form is configured to change in response to a stimulus, so as to redirect sweat and control an amount of sweat able to be transported to the sweat sensing component (102) via the sweat transport channel (104),
wherein the second sweat redirecting component (107) is positioned at an inlet of the sweat transport channel (104) so as to function as a valve such that, in a first configuration, the form of the second responsive material is such that the second sweat redirecting component (107) prevents the passage of sweat to the sweat sensing component (102) via the sweat transport channel (104); and, in a second configuration, the form of the second responsive material is such that the second sweat redirecting component (107) permits the passage of sweat to the sweat sensing component (102) via the sweat transport channel (104).

2. An apparatus (100) according to claim 1, wherein the stimulus comprises an electrical stimulus applied to the first responsive material and/or the second responsive material to bring about a change in the form of the first responsive material and/or the second responsive material, respectively; or a fluidic stimulus to bring about a change in the form of the first responsive material and/or the second responsive material when the fluidic stimulus comes into contact with the first responsive material and/or the second responsive material, respectively.

3. An apparatus (100) according to claim 1 or claim 2, wherein the stimulus comprises an electrical stimulus applied to the first responsive material and/or the second responsive material responsive to at least one of:
a parameter value of the sweat measured using the sweat sensing component (102) meeting or exceeding a parameter value threshold; and
a sweat rate of sweat from the sweat glands exceeding a sweat rate threshold.

4. An apparatus (100) according to claim 3, wherein the first responsive material and/or the second responsive material comprises at least one material selected from a group comprising: an electroactive polymer; and a piezoelectric material.

5. An apparatus (100) according to claim 3 or claim 4, further comprising: a sweat rate sensor to measure the sweat rate of sweat from the sweat glands.

6. An apparatus (100) according to any of claims 3 to 5, further comprising:
a processor configured to:
cause the electrical stimulus to be applied to the first responsive material and/or the second responsive material.

7. An apparatus (100) according to claim 1 or claim 2, wherein the stimulus comprises a fluidic stimulus to bring about a change in the form of the first responsive material and/or the second responsive material responsive to an amount of sweat contacting the first responsive material and/or the second responsive material exceeding a threshold amount, respectively.

8. An apparatus (100) according to claim 7, wherein the first responsive material and/or the second responsive material comprises a hydrogel.

9. An apparatus (100) according to any of the preceding claims, wherein the parameter relating to sweat comprises a parameter selected from a group comprising: a concentration of a substance in the sweat; and an osmolality of a substance in the sweat.

10. A method (800) of controlling the passage of sweat in an apparatus (100), the apparatus having a sweat sensing component (102), a sweat transport channel (104), a first sweat redirecting component (106) comprising a first responsive material and a second sweat redirecting component (107) comprising a second responsive material, the method comprising:
receiving (802), at a processor, data indicative of a parameter relating to sweat generated by sweat glands of a subject; and
causing (804) an electrical stimulus to be applied to the first responsive material and/or the second responsive material based on the received data;
wherein application of the electrical stimulus to the first responsive material causes a form of the first responsive material to change, thereby to redirect sweat and control an amount of sweat able to be transported to the sweat sensing component (102) via the sweat transport channel (104),
wherein application of the electrical stimulus to the second responsive material causes a form of the second responsive material to change, thereby to redirect sweat and control an amount of sweat able to be transported to the sweat sensing component (102) via the sweat transport channel (104),
wherein the first sweat redirecting component (106) is positioned at an outlet of the sweat transport channel (104), such that, in a first configuration, the form of the first responsive material is such that the first sweat redirecting component (106) prevents the passage of sweat through the outlet, and directs the passage of sweat to the sweat sensing component (102); and, in a second configuration, the form of the first responsive material is such that the first sweat redirecting component (106) permits the passage of sweat through the outlet, and
wherein the second sweat redirecting component (107) is positioned at an inlet of the sweat transport channel (104) so as to function as a valve such that, in a first configuration, the form of the second responsive material is such that the second sweat redirecting component (107) prevents the passage of sweat to the sweat sensing component (102) via the sweat transport channel (104); and, in a second configuration, the form of the second responsive material is such that the second sweat redirecting component (107) permits the passage of sweat to the sweat sensing component (102) via the sweat transport channel (104).

11. A method (800) according to claim 10, wherein causing an electrical stimulus to be applied to the first responsive material and/or the second responsive material comprises controlling a power source to supply a current or a voltage to the first responsive material and/or the second responsive material, respectively.

12. A system (1000) comprising:
an apparatus (100) according to any of claims 1 to 9; and
a processor (902) configured to:
cause an electrical stimulus to be applied to the first responsive material and/or the second responsive material.

## Patentansprüche

1. Einrichtung (100), umfassend:
eine Schweißsensorkomponente (102) zum Messen eines Parameters in Bezug auf von Schweißdrüsen einer Person erzeugten Schweiß;
einen Schweißtransportkanal (104); und
eine erste Schweißumleitungskomponente (106), die ein erstes ansprechendes Material umfasst, dessen Form konfiguriert ist, um sich als Reaktion auf einen Reiz zu ändern, um Schweiß umzuleiten und eine Schweißmenge zu kontrollieren, die imstande ist, über den Schweißtransportkanal (104) zur Schweißsensorkomponente (102) transportiert zu werden;
wobei die erste Schweißumleitungskomponente (106) an einem Auslass des Schweißtransportkanals (104) positioniert ist, sodass in einer ersten Konfiguration die Form des ersten ansprechenden Materials derart ist, dass die erste Schweißumleitungskomponente (106) den Durchtritt von Schweiß durch den Auslass verhindert, und den Durchtritt von Schweiß zur Schweißsensorkomponente (102) leitet; und wobei in einer zweiten Konfiguration die Form des ersten ansprechenden Materials derart ist, dass die erste Schweißumleitungskomponente (106) den Durchtritt von Schweiß durch den Auslass zulässt, **dadurch gekennzeichnet, dass**:
die Einrichtung eine zweite Schweißumleitungskomponente (107) umfasst, die ein zweites ansprechendes Material umfasst, dessen Form konfiguriert ist, um sich als Reaktion auf einen Reiz zu ändern, um Schweiß umzuleiten und eine Schweißmenge zu kontrollieren, die imstande ist, über den Schweißtransportkanal (104) zur Schweißsensorkomponente (102) transportiert zu werden,
wobei die zweite Schweißumleitungskomponente (107) an einem Einlass des Schweißtransportkanals (104) positioniert ist, um als Ventil zu fungieren, sodass in einer ersten Konfiguration die Form des zweiten ansprechenden Materials derart ist, dass die zweite Schweißumleitungskomponente (107) den Durchtritt von Schweiß zu der Schweißsensorkomponente (102) über den Schweißtransportkanal (104) verhindert; und wobei in einer zweiten Konfiguration die Form des zweiten ansprechenden Materials derart ist, dass die zweite Schweißumleitungskomponente (107) den Durchtritt von Schweiß zu der Schweißsensorkomponente (102) über den Schweißtransportkanal (104) zulässt.

2. Einrichtung (100) nach Anspruch 1, wobei der Reiz einen elektrischen Reiz, der auf das erste ansprechende Material und/oder das zweite ansprechende Material angewendet wird, um jeweils eine Änderung der Form des ersten ansprechenden Materials und/oder des zweiten ansprechenden Materials herbeizuführen; oder einen fluidischen Reiz umfasst, um eine Änderung der Form des ersten ansprechenden Materials und/oder des zweiten ansprechenden Materials herbeizuführen, wenn der fluidische Reiz jeweils mit dem ersten ansprechenden Material und/oder dem zweiten ansprechenden Material in Kontakt kommt.

3. Einrichtung (100) nach Anspruch 1 oder Anspruch 2, wobei der Reiz einen elektrischen Reiz umfasst, der auf das erste ansprechende Material angewendet wird und/oder das zweite ansprechende Material auf mindestens eines anspricht von:
einem Parameterwert des Schweißes, der unter Verwendung der Schweißsensorkomponente (102) gemessen wird, der eine Parameterwertschwelle erreicht oder überschreitet; und
einer Schweißrate von Schweiß aus den Schweißdrüsen, die eine Schweißratenschwelle überschreitet.

4. Einrichtung (100) nach Anspruch 3, wobei das erste ansprechende Material und/oder das zweite ansprechende Material mindestens ein Material umfasst, das aus einer Gruppe ausgewählt ist, umfassend: ein elektroaktives Polymer; und ein piezoelektrisches Material.

5. Einrichtung (100) nach Anspruch 3 oder Anspruch 4, weiter umfassend:
ein Schweißratensensor, um die Schweißrate von Schweiß aus den Schweißdrüsen zu messen.

6. Einrichtung (100) nach einem der Ansprüche 3 bis 5, weiter umfassend:
einen Prozessor, der konfiguriert ist, um zu:
bewirken, dass der elektrische Reiz auf das erste ansprechende Material und/oder das zweite ansprechende Material angewendet wird.

7. Einrichtung (100) nach Anspruch 1 oder Anspruch 2, wobei der Reiz einen fluidischen Reiz umfasst, um eine Änderung der Form des ersten ansprechenden Materials und/oder des zweiten ansprechenden Materials herbeizuführen, die auf eine Schweißmenge ansprechen, die jeweils das erste ansprechende Material und/oder das zweite ansprechende Material kontaktiert, die eine Schwellenmenge überschreitet.

8. Einrichtung (100) nach Anspruch 7, wobei das erste ansprechende Material und/oder das zweite ansprechende Material ein Hydrogel umfasst.

9. Einrichtung (100) nach einem der vorstehenden Ansprüche, wobei der sich auf den Schweiß beziehende Parameter einen Parameter umfasst, der aus einer Gruppe ausgewählt ist, umfassend: eine Konzentration einer Substanz in dem Schweiß; und eine Osmolalität einer Substanz in dem Schweiß.

10. Verfahren (800) zum Kontrollieren des Schweißdurchtritts in einer Einrichtung (100), wobei die Einrichtung eine Schweißsensorkomponente (102), einen Schweißtransportkanal (104), eine erste Schweißumleitungskomponente (106), die ein erstes ansprechendes Material umfasst, und eine zweite Schweißumleitungskomponente (107), die ein zweites ansprechendes Material umfasst, aufweist, wobei das Verfahren umfasst:
Empfangen (802) von Daten an einem Prozessor, die einen Parameter in Bezug auf von Schweißdrüsen einer Person erzeugten Schweiß angeben; und
Bewirken (804), dass basierend auf den empfangenen Daten ein elektrischer Reiz auf das erste ansprechende Material und/oder das zweite ansprechende Material angewendet wird;
wobei Anwendung des elektrischen Reizes auf das erste ansprechende Material bewirkt, dass sich eine Form des ersten ansprechenden Materials ändert, wodurch Schweiß umgeleitet und eine Schweißmenge kontrolliert wird, die imstande ist, über den Schweißtransportkanal (104) zur Schweißsensorkomponente (102) transportiert zu werden,
wobei Anwendung des elektrischen Reizes auf das zweite ansprechende Material bewirkt, dass sich eine Form des zweiten ansprechenden Materials ändert, wodurch Schweiß umgeleitet und eine Schweißmenge kontrolliert wird, die imstande ist, über den Schweißtransportkanal (104) zur Schweißsensorkomponente (102) transportiert zu werden,
wobei die erste Schweißumleitungskomponente (106) an einem Auslass des Schweißtransportkanals (104) positioniert ist, sodass in einer ersten Konfiguration die Form des ersten ansprechenden Materials derart ist, dass die erste Schweißumleitungskomponente (106) den Durchtritt von Schweiß durch den Auslass verhindert, und den Durchtritt von Schweiß zur Schweißsensorkomponente (102) leitet; und wobei in einer zweiten Konfiguration die Form des ersten ansprechenden Materials derart ist, dass die erste Schweißumleitungskomponente (106) den Durchtritt von Schweiß durch den Auslass zulässt, und
wobei die zweite Schweißumleitungskomponente (107) an einem Einlass des Schweißtransportkanals (104) positioniert ist, um als Ventil zu fungieren, sodass in einer ersten Konfiguration die Form des zweiten ansprechenden Materials derart ist, dass die zweite Schweißumleitungskomponente (107) den Durchtritt von Schweiß zu der Schweißsensorkomponente (102) über den Schweißtransportkanal (104) verhindert; und wobei in einer zweiten Konfiguration die Form des zweiten ansprechenden Materials derart ist, dass die zweite Schweißumleitungskomponente (107) den Durchtritt von Schweiß zu der Schweißsensorkomponente (102) über den Schweißtransportkanal (104) zulässt.

11. Verfahren (800) nach Anspruch 10, wobei Bewirken, dass ein elektrischer Reiz auf das erste ansprechende Material und/oder das zweite ansprechende Material angewendet wird, Kontrollieren einer Leistungsquelle umfasst, um jeweils dem ersten ansprechenden Material und/oder dem zweiten ansprechenden Material einen Strom oder eine Spannung zuzuführen.

12. System (1000), umfassend:
eine Einrichtung (100) nach einem der Ansprüche 1 bis 9; und
einen Prozessor (902), der konfiguriert ist, um zu:
bewirken, dass ein elektrischer Reiz auf das erste ansprechende Material und/oder das zweite ansprechende Material angewendet wird.

## Revendications

1. Appareil (100) comprenant :
un composant de détection de transpiration (102) pour mesurer un paramètre relatif à de la transpiration générée par des glandes sudoripares d'un sujet ;
un canal de transport de transpiration (104) ; et
un premier composant de redirection de transpiration (106) comprenant un premier matériau sensible dont la forme est configurée pour changer en réponse à un stimulus, de manière à rediriger de la transpiration et à réguler une quantité de transpiration apte à être transportée vers le composant de détection de transpiration (102) par l'intermédiaire du canal de transport de transpiration (104) ;
dans lequel le premier composant de redirection de transpiration (106) est positionné à une sortie du canal de transport de transpiration (104), de sorte que, dans une première configuration, la forme du premier matériau sensible soit telle que le premier composant de redirection de transpiration (106) empêche le passage de transpiration à travers la sortie, et dirige le passage de transpiration vers le composant de détection de transpiration (102) ; et, dans une seconde configuration, la forme du premier matériau sensible est telle que le premier composant de redirection de transpiration (106) permette le passage de transpiration à travers la sortie, **caractérisé en ce que** :
l'appareil comprend un second composant de redirection de transpiration (107) comprenant un second matériau sensible dont la forme est configurée pour changer en réponse à un stimulus, de manière à rediriger de la transpiration et à réguler une quantité de transpiration apte à être transportée vers le composant de détection de transpiration (102) par l'intermédiaire du canal de transport de transpiration (104),
dans lequel le second composant de redirection de transpiration (107) est positionné à une entrée du canal de transport de transpiration (104) de manière à fonctionner comme une vanne de sorte que, dans une première configuration, la forme du second matériau sensible soit telle que le second composant de redirection de transpiration (107) empêche le passage de transpiration vers le composant de détection de transpiration (102) par l'intermédiaire du canal de transport de transpiration (104) ; et, dans une seconde configuration, la forme du second matériau sensible est telle que le second composant de redirection de transpiration (107) permette le passage de transpiration vers le composant de détection de transpiration (102) par l'intermédiaire du canal de transport de transpiration (104).

2. Appareil (100) selon la revendication 1, dans lequel le stimulus comprend un stimulus électrique appliqué au premier matériau sensible et/ou au second matériau sensible pour provoquer un changement de forme du premier matériau sensible et/ou du second matériau sensible, respectivement ; ou un stimulus fluidique pour provoquer un changement de forme du premier matériau sensible et/ou du second matériau sensible lorsque le stimulus fluidique entre en contact avec le premier matériau sensible et/ou le second matériau sensible, respectivement.

3. Appareil (100) selon la revendication 1 ou la revendication 2, dans lequel le stimulus comprend un stimulus électrique appliqué au premier matériau sensible et/ou au second matériau sensible en réponse à au moins un parmi :
une valeur de paramètre de la transpiration mesurée en utilisant le composant de détection de transpiration (102) atteignant ou dépassant un seuil de valeur de paramètre ; et
un taux de transpiration de la transpiration provenant des glandes sudoripares dépassant un seuil de taux de transpiration.

4. Appareil (100) selon la revendication 3, dans lequel le premier matériau sensible et/ou le second matériau sensible comprend au moins un matériau sélectionné à partir d'un groupe comprenant : un polymère électroactif ; et un matériau piézoélectrique.

5. Appareil (100) selon la revendication 3 ou la revendication 4, comprenant en outre :
un capteur de taux de transpiration pour mesurer le taux de transpiration de la transpiration provenant des glandes sudoripares.

6. Appareil (100) selon l'une quelconque des revendications 3 à 5, comprenant en outre :
un processeur configuré pour :
provoquer l'application du stimulus électrique au premier matériau sensible et/ou au second matériau sensible.

7. Appareil (100) selon la revendication 1 ou la revendication 2, dans lequel le stimulus comprend un stimulus fluidique pour provoquer un changement de forme du premier matériau sensible et/ou du second matériau sensible en réponse à une quantité de transpiration entrant en contact avec le premier matériau sensible et/ou le second matériau sensible dépassant une quantité seuil, respectivement.

8. Appareil (100) selon la revendication 7, dans lequel le premier matériau sensible et/ou le second matériau sensible comprend un hydrogel.

9. Appareil (100) selon l'une quelconque des revendications précédentes, dans lequel le paramètre relatif à la transpiration comprend un paramètre sélectionné à partir d'un groupe comprenant : une concentration d'une substance dans la transpiration ; et une osmolalité d'une substance dans la transpiration.

10. Procédé (800) de régulation du passage de transpiration dans un appareil (100), l'appareil présentant un composant de détection de transpiration (102), un canal de transport de transpiration (104), un premier composant de redirection de transpiration (106) comprenant un premier matériau sensible et un second composant de redirection de transpiration (107) comprenant un second matériau sensible, le procédé comprenant :
la réception (802), au niveau d'un processeur, de données indiquant un paramètre relatif à de la transpiration générée par les glandes sudoripares d'un sujet ; et
le fait de provoquer (804) l'application d'un stimulus électrique au premier matériau sensible et/ou au second matériau sensible sur la base des données reçues ;
dans lequel l'application du stimulus électrique au premier matériau sensible provoque un changement de forme du premier matériau sensible, pour ainsi rediriger la transpiration et réguler une quantité de transpiration apte à être transportée vers le composant de détection de transpiration (102) par l'intermédiaire du canal de transport de transpiration (104),
dans lequel l'application du stimulus électrique au second matériau sensible provoque une modification de la forme du second matériau sensible, pour ainsi rediriger la transpiration et réguler une quantité de transpiration apte à être transportée vers le composant de détection de transpiration (102) par l'intermédiaire du canal de transport de transpiration (104),
dans lequel le premier composant de redirection de transpiration (106) est positionné à une sortie du canal de transport de transpiration (104), de sorte que, dans une première configuration, la forme du premier matériau sensible soit telle que le premier composant de redirection de transpiration (106) empêche le passage de transpiration à travers la sortie, et dirige le passage de transpiration vers le composant de détection de transpiration (102) ; et, dans une seconde configuration, la forme du premier matériau sensible est telle que le premier composant de redirection de transpiration (106) permette le passage de transpiration à travers la sortie, et
dans lequel le second composant de redirection de transpiration (107) est positionné à une entrée du canal de transport de transpiration (104) de manière à fonctionner comme une vanne de sorte que, dans une première configuration, la forme du second matériau sensible soit telle que le second composant de redirection de transpiration (107) empêche le passage de transpiration vers le composant de détection de transpiration (102) par l'intermédiaire du canal de transport de transpiration (104) ; et dans une seconde configuration, la forme du second matériau sensible est telle que le second composant de redirection de transpiration (107) permette le passage de transpiration vers le composant de détection de transpiration (102) par l'intermédiaire du canal de transport de transpiration (104).

11. Procédé (800) selon la revendication 10, dans lequel le fait de provoquer l'application d'un stimulus électrique au premier matériau sensible et/ou au second matériau sensible comprend la commande d'une source d'énergie pour fournir un courant ou une tension au premier matériau sensible et/ou au second matériau sensible, respectivement.

12. Système (1000) comprenant :
un appareil (100) selon l'une quelconque des revendications 1 à 9 ; et
un processeur (902) configuré pour :
provoquer l'application d'un stimulus électrique au premier matériau sensible et/ou au second matériau sensible.
